# EUROPEAN PATENT SPECIFICATION

(11) **EP 1 944 029 B1**
(45) Date of publication and mention of the grant of the patent: **21.12.2011**
(21) Application number: 06822589.5
(22) Date of filing: 30.10.2006
(51) Int. Cl.: A61K 31/47, A61K 9/16, A61K 9/28, A61K 47/02, A61K 47/20, A61P 3/06, A61K 9/20, A61K 9/50

(54) **PHARMACEUTICAL PREPARATION HAVING EXCELLENT PHOTOSTABILITY**
PHARMAZEUTISCHE ZUBEREITUNG MIT HERVORRAGENDER PHOTOSTABILITÄT
PREPARATION PHARMACEUTIQUE AYANT UNE PHOTOSTABILITE EXCELLENTE

(30) Priority: 31.10.2005 JP 2005316758
(43) Date of publication of application: 16.07.2008
(73) Proprietor: Kowa Company, Ltd., Nagoya-shi, Aichi-ken 460-8625 (JP); Nissan Chemical Industries, Ltd., Chiyoda-ku, Tokyo 101-0054 (JP)
(72) Inventor: KOZAKI, Masato, Fuji-shi Shizuoka 417-8650 (JP); TANIZAWA, Yoshio, Fuji-shi Shizuoka 417-8650 (JP); KAWASHIMA, Hiroyuki, Fuji-shi Shizuoka 417-8650 (JP)
(74) Representative: Blodig, Wolfgang
(86) International application number: PCT/JP2006/321632
(87) International publication number: WO 2007/052592

(56) References cited:
- EP-A1- 0 901 787
- WO-A1-2004/010980
- WO-A2-2004/071403
- WO-A2-2004/071403
- JP-A- 48 028 621
- JP-A- 55 022 645
- JP-A- 58 109 415
- JP-A- 2000 191 516
- JP-A- 2000 191 516
- JP-A- 2003 104 887

## Description

### Field of the Invention

The present invention relates to a pharmaceutical preparation of high photostability which contains pitavastatin, which is an HMG-CoA reductase inhibitor, a salt of pitavastatin, or an ester of pitavastatin.

### Background Art

Pitavastatin, a salt thereof, and an ester thereof (hereinafter these species are collectively referred to as pitavastatin compounds) exhibit an excellent HMG-CoA reductase inhibitory effect and, therefore, are useful drugs for the treatment of hyperlipemia, hypercholesterolemia, atherosclerosis, etc. (JP-A-1-279866) In order to mitigate a bitter taste of pitavastatin, commercial solid pharmaceutical preparations thereof are produced by incorporating pitavastatin into a base material and coating the drug base with film.

### Disclosure of the Invention

### Problems to be Solved by the Invention

Preceding studies conducted by the present inventors have revealed that pitavastatin compounds had poor stability against light; i.e., poor photostability. Actually, as mentioned hereinbelow, pitavastatin compounds are decomposed by light to form decomposition products including a ring-closed form 1, a ring-closed form 2, and a 5-keto form. On the basis of the studies, the inventors recognized that a base material of a pitavastatin pharmaceutical preparation must also be imparted with stability against light through a certain treatment, since pitavastatin contained in the pharmaceutical preparation (e.g., in the form of scored tablet, which is designed to be taken after being split) decomposes when a broken surface is exposed to light. Generally, scored tablets facilitate optimum dose control of patients and assure flexibility in prescription. Therefore, development of a scored-tablet-form pitavastatin pharmaceutical preparation is demanded in view of improving patient's compliance.

A drug instable against light is incorporated into a solid pharmaceutical preparation, through a known technique such as encapsulation or coating, so as to enhance photostability of the preparation. For example, JP-A-2002-212104 discloses a light-resistant pharmaceutical preparation coated with a light protecting agent such as talc or barium sulfate; Pamphlet of International Publication No. 97/39752 discloses a Sertindole-containing pharmaceutical preparation in which base granules are coated with shells containing titanium oxide; JP-A-2003-104887 discloses an aranidipine-containing composition having a solid-dispersed pharmaceutical preparation having a surface sprayed with a light-protecting agent and a colorant.
Instead of coating, one known technique of enhancing photostability is through incorporation of a specific substance into a base material of a solid pharmaceutical preparation. Specifically, JP-A-2000-7583 discloses an agent which has a lipid-soluble drug having poor stability against light, where the photostability of the lipid-soluble drug is improved by incorporation of a yellow or a red dye. JP-A-2000-191516 discloses an agent having improved photostability, in which a drug having poor stability against light is improved in its photostability by incorporation of a colorant. The patent document also discloses that, when the agent further contains titanium oxide, the effect of enhancing photostability can be further potentiated. Other relevant prior art comprises JP 200191516 and WO 2004071403.
However, hitherto, only film coating of tablets has been known as a technique for preventing photo-decomposition of pitavastatin compounds, and currently, no pharmaceutical preparation containing the pitavastatin compound suitable to be served in the form of scored tablets, granules, or fine granules is known, nor is a production method therefor.

Under such circumstances, an object of the present invention is to provide a pharmaceutical preparation containing the pitavastatin compound and having excellent photostability.

### Means for Solving the Problems

As mentioned above, the present inventors previously found that pitavastatin compounds change their chemical structures under exposure to light, thereby forming decomposition products represented by the following formulas:

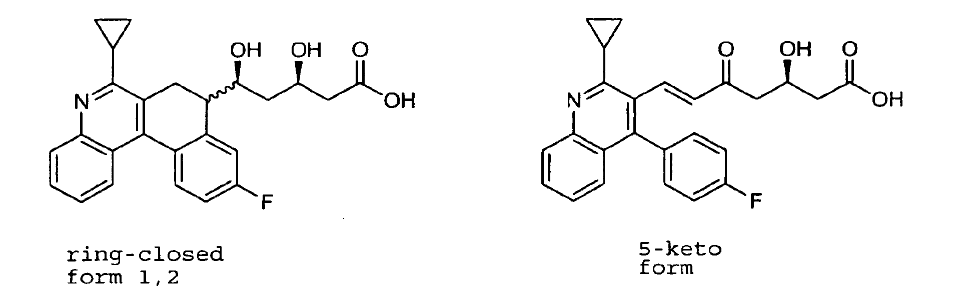

More specifically, irradiation of pitavastatin compounds with light was found to form the following three decomposition products: (3R,5S)-5-[(8R)-6-cyclopropyl-7,8-dihydro-10-fluorobenzo[k]phenanthridin-8-il]-3,5-dihydroxypentanoic acid (hereinafter referred to as ring-closed form 1), (3R,5S)-5-[(8S)-6-cyclopropyl-7,8-dihydro-10-fluorobenzo[k]phenanthridine-8-il]-3,5-dihydroxypentanoic acid (hereinafter referred to as ring-closed form 2), and (3R,6E)-7-[2-cyclopropyl-4-(4-fluorophenyl)-3-quinolyl]-3-hydroxy-5-oxo-6-heptenoic acid (referred to as 5-keto form).
When the above compounds are formed, the pitavastatin level of a pharmaceutical preparation cannot be maintained, possibly failing to attain sufficient treatment effects.

In order to solve the above problem, the present inventors have carried out extensive studies on the approach for preventing formation of the above three decomposition products, and have found that use of titanium oxide, which is widely employed as a light-protecting agent, can prevent formation of ring-closed forms, but cannot sufficiently prevent formation of 5-keto form. Through further studies, the inventors have found that incorporation of a colorant having a maximum absorption wavelength of 400 nm to 500 nm into a pharmaceutical preparation successfully prevents formation of 5-keto form. The inventors have further found that use in combination of titanium oxide and a colorant having a maximum absorption wavelength of 400 nm to 500 nm can prevent formation of both pitavastatin ring-closed forms and 5-keto form in a pharmaceutical preparation. The present invention has been accomplished on the basis of these findings.

Accordingly, in a first aspect of the present invention, there is provided a pharmaceutical preparation containing a pitavastatin compound, titanium oxide, and a colorant having a maximum absorption wavelength of 400 nm to 500 nm.
The present invention also provides a pharmaceutical preparation comprising granules containing a pitavastatin compound, titanium oxide, and a colorant having a maximum absorption wavelength of 400 nm to 500 nm.
The present invention also provides a pharmaceutical preparation comprising granules including
granulated particles containing a pitavastatin compound and a colorant having a maximum absorption wavelength of 400 nm to 500 nm, and
a coating shell which contains titanium oxide and which covers each of the granulated particles.
The present invention also provides a pharmaceutical preparation comprising granules including
granulated particles containing a pitavastatin compound,
an intermediate shell which contains a colorant having a maximum absorption wavelength of 400 nm to 500 nm and which covers each of the granulated particles, and
a coating shell which contains titanium oxide and which covers the intermediate shell.
The present invention also provides a pharmaceutical preparation comprising granules including
granulated particles containing a pitavastatin compound and titanium oxide, and
a coating shell which contains a colorant having a maximum absorption wavelength of 400 nm to 500 nm and which covers each of the granulated particles.
The present invention also provides a pharmaceutical preparation comprising granules including
granulated particles containing a pitavastatin compound,
an intermediate shell which contains titanium oxide and which covers each of the granulated particles, and
a coating shell which contains a colorant having a maximum absorption wavelength of 400 nm to 500 nm and which covers the intermediate shell.

In a second aspect of the present invention, there is provided a method for producing granules comprising producing granulated particles each containing a pitavastatin compound, titanium oxide, and a colorant having a maximum absorption wavelength of 400 nm to 500 nm.
The present invention also provides a method for producing granules comprising
producing granulated particles each containing a pitavastatin compound and a colorant having a maximum absorption wavelength of 400 nm to 500 nm, and
subsequently, coating each of the granulated particles with a coating liquid containing titanium oxide.
The present invention also provides a method for producing granules comprising
producing granulated particles each containing a pitavastatin compound,
subsequently, coating each of the granulated particles with a coating liquid containing a colorant having a maximum absorption wavelength of 400 nm to 500 nm, and
subsequently, coating the coated particle with a coating liquid containing titanium oxide.
The present invention also provides a method for producing granules comprising
producing granulated particles each containing a pitavastatin compound and titanium oxide, and
subsequently, coating each of the granulated particles with a coating liquid containing a colorant having a maximum absorption wavelength of 400 nm to 500 nm.
The present invention also provides a method for producing granules comprising
producing granulated particles each containing a pitavastatin compound,
subsequently, coating each of the granulated particles with a coating liquid containing titanium oxide, and
subsequently, coating the coated particle with a coating liquid containing a colorant having a maximum absorption wavelength of 400 nm to 500 nm.

### Effects of the Invention

According to the present invention, photostability of the pitavastatin compound contained in a pharmaceutical preparation is maintained. Therefore, photo-decomposition of the pitavastatin compound contained in, for example, scored tablets, which are split upon use, can be suppressed, whereby the highly effective treatment of hyperlipemia and hypercholesterolemia can be realized.

### Mode for Carrying out the Invention

The pitavastatin compound employed in the present invention include pitavastatin ((3R,5S,6E)-7-[2-cyclopropyl-4-(4-fluorophenyl)-3-quinolyl]-3,5-dihydroxy-6-heptenoic acid): USP No. 5856336 and JP-A-1-279866), a salt of pitavastatin, and an ester of pitavastatin (including lactone-ring-formed species). In addition, hydrates thereof and solvates thereof with a pharmaceutically acceptable solvent are also included. As mentioned above, pitavastatin compounds are known to be served as an excellent HMG-CoA reductase inhibitor and a useful treatment drug for hyperlipemia, hypercholesterolemia, etc.
Examples of the pitavastatin salt include salts of alkali metal such as sodium or potassium, salts of alkaline earth metal such as calcium or magnesium, salts of organic amine such as phenethylamine, and ammonium salts. Examples of the ester of pitavastatin includes C1 to C6 lower alkyl esters, and specific examples include methyl esters, ethyl esters, i-propyl esters, and n-propyl esters. Among these, pitavastatin salts are preferred, with a calcium salt thereof being particularly preferred.
Pitavastatin compounds can be produced through any of the methods disclosed in USP No. 5,856,336 or JP-A-1-279866.

No particular limitation is imposed on the content of the pitavastatin compound of the pharmaceutical preparation of the present invention, and the content is preferably 0.01 to 15 mass% with respect to the total amount of the pharmaceutical preparation, more preferably 0.1 to 8 mass%, particularly preferably 0.5 to 4 mass%.

Titanium oxide employed in the present invention may have a rutile crystal-form or an anatase crystal-form. Examples of titanium oxide commercial products include titanium oxide NA-65 (rutile form, product of Toho Titanium) and titanium oxide A-HR (anatase form, product of TIOXIDE).
In order to prevent decomposition of the pitavastatin compound, the titanium oxide content of the pharmaceutical preparation of the present invention is preferably 0.01 to 25 mass% with respect to the total amount of the pharmaceutical preparation, more preferably 0.1 to 20 mass%, particularly preferably 0.5 to 15 mass%.

The colorant employed in the present invention has a maximum absorption wavelength of 400 nm to 500 nm in a UV absorption spectrum. Particularly, a colorant having an absorption maximum of 450 nm to 500 nm is preferred. Examples of the colorant include Food Yellow No. 5 (maximum absorption wavelength: 482±2 nm) and Food Yellow No. 4 (maximum absorption wavelength: 428±2 nm). Of these, Food Yellow No. 5 is particularly preferred. The maximum absorption wavelengths may be determined in the UV absorption spectra by means of a spectrophotometer (U-3010: product of Hitachi Corporation) or a similar device.

The colorant content of the pharmaceutical preparation of the present invention is preferably 0.001 to 4 mass% with respect to the total amount of the pharmaceutical preparation, more preferably 0.005 to 2 mass%, particularly preferably 0.01 to 1 mass%, from the viewpoint of prevention of decomposition of the pitavastatin compound.

As mentioned above, the pharmaceutical preparation of the present invention contains a pitavastatin compound, titanium oxide, and a colorant having a maximum absorption wavelength of 400 nm to 500 nm. No particular limitation is imposed on the drug form of the pharmaceutical preparation so long as the preparation is a peroral solid. Examples of the drug form include granules, fine granules, tablets, and capsules. The preparation may be a film-coated drug produced through a routine film coating method. The tablets may be rapid-release tablets or sustained-release tablets. Of these, rapid-release tablets are preferred.
Specifically, tablets may be produced by mixing the above ingredients with an optional pharmacologically acceptable carrier and pelletizing the resultant mixture.
The capsule-form drugs may be produced by filling capsules with the drug content. In order to facilitate breaking of tablets, each tablet may be provided with at least one score line (i.e., groove), thereby producing scored tablets, which can be split before use. According to the present invention, even when such scored tablets are split before use and the split surface of each tablet fragment is exposed to light, the tablets have been stabilized through the aforementioned procedure. In the present invention, scored tablets are particularly preferred.

In the case of, for example, tablets, the pharmaceutical preparation may be produced by admixing or granulating a pitavastatin compound and titanium oxide, or a pitavastatin compound and a colorant having a maximum absorption wavelength of 400 nm to 500 nm; pelletizing the admixture or granulated particles through a routine method; and, subsequently, coating the pellets with a coating liquid containing the colorant and/or titanium oxide. Alternatively, the encapsulated preparation may be produced by admixing or granulating a pitavastatin compound and titanium oxide, or a pitavastatin compound and the colorant; and filling capsules each containing the colorant and/or titanium oxide with the admixture or granulated particles.

Preferably, the pharmaceutical preparation of the present invention is obtained in the form of granules, which are then formed into encapsulated drugs, tablets, and drugs of other forms, from the viewpoint of maintaining the stability of the pitavastatin compound.

Examples of the pharmaceutical preparation in the form of granules of the present invention include (1) granulated particles (granules) containing a pitavastatin compound, titanium oxide, and a colorant having a maximum absorption wavelength of 400 nm to 500 nm; (2) granules including granulated particles containing a pitavastatin compound and a colorant having a maximum absorption wavelength of 400 nm to 500 nm, and a coating shell which contains titanium oxide and which covers each of the granulated particles; (3) granules including granulated particles containing a pitavastatin compound, an intermediate shell which contains a colorant having a maximum absorption wavelength of 400 nm to 500 nm and which covers each of the granulated particles, and a coating shell which contains titanium oxide and which covers the intermediate shell; (4) granules including granulated particles containing a pitavastatin compound and titanium oxide, and a coating shell which contains a colorant having a maximum absorption wavelength of 400 nm to 500 nm and which covers each of the granulated particles; and (5) granules including granulated particles containing a pitavastatin compound, an intermediate shell which contains titanium oxide and which covers each of the granulated particles, and a coating shell which contains a colorant having a maximum absorption wavelength of 400 nm to 500 nm and which covers the intermediate shell.

Among these granules, granules (1) can be produced through granulating a pitavastatin compound, titanium oxide, and a colorant having a maximum absorption wavelength of 400 nm to 500 nm.
Granules (2) can be produced through granulating a pitavastatin compound with a colorant having a maximum absorption wavelength of 400 nm to 500 nm, and subsequently, coating each of the granulated particles with a coating liquid containing titanium oxide.
Granules (3) can be produced through granulating a pitavastatin compound, subsequently, coating each of the granulated particles with a coating liquid containing a colorant having a maximum absorption wavelength of 400 nm to 500 nm, and subsequently, coating the coated particle with a coating liquid containing titanium oxide.
Granules (4) can be produced through granulating a pitavastatin compound with titanium oxide, and subsequently, coating each of the granulated particles with a coating liquid containing a colorant having a maximum absorption wavelength of 400 nm to 500 nm.
Granules (5) can be produced through granulating a pitavastatin compound, subsequently, coating each of the granulated particles with a coating liquid containing titanium oxide, and subsequently, coating the coated particle with a coating liquid containing a colorant having a maximum absorption wavelength of 400 nm to 500 nm.

Granulation may be performed through known techniques such as fluidized bed granulation, tumbling granulation, agitation granulation, and spray granulation. Coating may be performed through methods such as pan-coating, tumbling coating, and fluidization coating. Fluidized bed granulation is particularly preferred, since granulation, coating, and drying can be performed in one single apparatus.

The granules of the invention may contain a pharmacologically acceptable carrier in accordance with needs. Such a carrier may be appropriately added to the granules through mixing with at least one component selected from among a pitavastatin compound, titanium oxide, and a colorant having a maximum absorption wavelength of 400 nm to 500 nm.

The aforementioned granules themselves may be employed as a granule-form pharmaceutical preparation. Alternatively, the granules may be appropriately mixed with a pharmacologically acceptable carrier, and the mixture may be formed into a variety shape of pharmaceutical preparations through a routine method.

In the present invention, examples of the carrier which can be mixed with the granules or with at least one component selected from among a pitavastatin compound, titanium oxide, and a colorant having a maximum absorption wavelength of 400 nm to 500 nm include vehicles (bulking agent) such as lactose, corn starch, modified corn starch, wood cellulose, microcrystalline cellulose, and calcium carbonate; binders such as hydroxypropyl cellulose, hydroxypropylmethyl cellulose, polyvinylpyrroridone, and poly(vinyl alcohol) (partial-saponified product); disintegrants such as low-substituted hydroxypropyl cellulose, carmelose, carboxystarch sodium, carmelose calcium, corn starch, partial-α-starch, cross-carmelose sodium, and cross-povidone; lubricants such as magnesium stearate, stearic acid, palmitic acid, calcium stearate, and talc. These carriers may be used singly or in combination. In accordance with needs, other ingredients such as a sweetener, a flavoring agent, a colorant, and perfume may be incorporated into the granules.

The following other additives may also be employed: sugars such as lactose, fructose, glucose, saccharose, maltose, sorbitol, xylitol, maltitol, mannitol, trehalose, cyclodextrin, erythritol, reduced palatinose, and lactitol; cellulose derivatives such as methyl cellulose, ethyl cellulose, hydroxypropyl cellulose , hydroxypropylmethyl cellulose, and low-substituted hydroxypropyl cellulose; polyvinyl derivatives such as polyvinylpyrrolidone, polyvinyl acetal diethylaminoacetate, and polyvinyl alcohol (partially saponified products); alkylene oxide polymers such as polyethylene glycol and polypropylene glycol; fats and oils such as sucrose fatty acid esters and polyoxyl 40 stearate.

To the pharmaceutical preparation of the present invention, preferably added is a basic substance which can elevate the pH of aqueous solution or aqueous dispersion of the granules to 6.8 or higher, particularly 6.8 to 7.8, so as to enhance time-dependent stability of the pitavastatin compound. Examples of the basic substance include acid-suppressors such as magnesium metasilicate aluminate, magnesium silicate aluminate, magnesium aluminate, dry aluminum hydroxide, synthetic hydrotalcite, synthetic aluminum silicate, magnesium carbonate, precipitated calcium carbonate, magnesium oxide, aluminum hydroxide, and sodium hydrogen carbonate; and pH-modifiers such as L-arginine, sodium phosphate, disodium hydrogen phosphate, sodium dihydrogen phosphate, potassium phosphate, dipotassium hydrogen phosphate, potassium dihydrogen phosphate, disodium citrate, sodium succinate, ammonium chloride, and sodium benzoate. Of these, magnesium metasilicate aluminate, L-arginine, and dipotassium hydrogen phosphate are particularly preferably used.
As used herein, the "pH" refers to that of a 5 w/v% (on the basis of unit dose) suspension of the pharmaceutical preparation of the present invention.

Examples of ingredients of the coating liquid employed in the present invention for covering granulated particles or providing coating film on pharmaceutical preparation grains include bio-degradable polymers, cellulose derivatives, (meth)acrylic (co)polymers, alkylene oxide polymers, fats and oils, silicones, chitin, chitosan, casein, tragacanth gum, guar gum, gellan gum, and acacia.
Examples of the biodegradable polymers include poly(lactic acid), poly(glycolic acid), poly(hydroxylactic acid), poly-α-cyanoacrylate esters, polyorthoesters, poly(amino acid), and gelatin.

Examples of the cellulose derivatives include methyl cellulose, ethyl cellulose, propyl cellulose, hydroxymethyl cellulose, hydroxyethyl cellulose, hydroxypropyl cellulose, hydroxypropylmethyl cellulose, carboxymethyl cellulose, carboxyethyl cellulose, carboxypropyl cellulose, cellulose acetylphthalate, cellulose diacetylphthalate, cellulose triacetylphthalate, polyoxyethyl cellulose phthalate, hydroxyethyl cellulose phthalate, hydroxypropyl cellulose phthalate, acetyl cellulose, and salts thereof.

Examples of the (meth)acrylic (co)polymers include ethyl acrylate-methyl methacrylate-trimethylethylammonium chloride methacrylate copolymer, butyl methacrylate-dimethylaminoethyl methacrylate copolymer, acrylic acid polymer, methyl acrylate polymer, and dimethylaminoethyl ether methacrylate polymer.

Examples of the alkylene oxide polymers include polyethylene glycol and polypropylene glycol.

Examples of the fats and oils include hardened oil, monoglycerides, triglycerides, wax, higher fatty acids, sucrose fatty acid esters, and higher fatty acid glycerin esters.
Examples of the silicones include silicones such as dimethylpolysiloxane, methylpolysiloxane, and silicone oil; dimethylpolysiloxane-silicon dioxide mixtures; silicone defoaming agents; and dimethylpolysiloxane mixtures such as silicone resin emulsion.

The coating liquid may further contain a sustained-release ingredient. Examples of the sustained-release ingredient include bio-degradable polymers, starches, dextrans, cellulose derivatives, (meth)acrylic (co)polymers, alkylene oxide polymers, fats and oils, carragheenan, chitin, chitosan, casein, tragacanth gum, guar gum, gellan gum, paraffins, silicones, acacia, poly(glutamic acid), poly(asprartic acid), poly(lysine), poly(arginine), alginic acid, pectic acid, and xanthane gum.

Examples of the biodegradable polymers include poly(lactic acid), poly(glycolic acid), poly(hydroxylactic acid), poly-α-cyanoacrylate esters, polyorthoesters, poly(amino acid), gelatin, collagen, chondroitin sulfate, hyaluronic acid, albumin, casein, globulin, and gluten.

Examples of the starches include α-amylostarch, gelatinized starch, carboxymethyl starch, carboxyethyl starch, phosphated starch, acid-treated starch, oxidized starch, dialdehyde starch, thin-boiling starch, and dextrin.
Examples of the dextrans include dextran, dextran sulfate, and carboxymethyl dextran.

Examples of the cellulose derivatives include methyl cellulose, ethyl cellulose, propyl cellulose, hydroxymethyl cellulose, hydroxyethyl cellulose, hydroxypropyl cellulose, carboxyethyl cellulose, carboxypropyl cellulose, cellulose acetylphthalate, cellulose diacetylphthalate, cellulose triacetylphthalate, polyoxyethyl cellulose phthalate, hydroxyethyl cellulose phthalate, hydroxypropyl cellulose phthalate, acetyl cellulose, and salts thereof.

Examples of the (meth)acrylic (co)polymers include ethyl acrylate-methyl methacrylate-trimethylethylammonium chloride methacrylate copolymer, butyl methacrylate-dimethylaminoethyl methacrylate copolymer, acrylic acid polymer, methyl acrylate polymer, and dimethylaminoethyl ether methacrylate polymer.

Examples of the alkylene oxide polymers include polyethylene glycol and polypropylene glycol.

Examples of the fats and oils include hardened oil, hardened castor oil, olive oil, monoglycerides, triglycerides, wax, higher fatty acids, sucrose fatty acid esters, higher alcohols, and higher fatty acid glycerin esters.

Examples of the silicones include silicones such as dimethylpolysiloxane, methylpolysiloxane, and silicone oil; dimethylpolysiloxane-silicon dioxide mixtures; silicone defoaming agents; and dimethylpolysiloxane mixtures such as silicone resin emulsion.

The does of the pharmaceutical preparation of the present invention is appropriately predetermined in accordance with the body weight, age, sex, type of disorder, and symptom of patients. The preparation of the invention may be administered in a dose, as reduced to a pitavastatin compound, 0.1 to 40 mg per day, preferably 1 to 20 mg per day in a single or divided manner.

### EXAMPLES

The present invention will next be described in more detail by way of examples.

### Example 1

Pitavastatin calcium (200 g), magnesium metasilicate aluminate (250 g), lactose (4,778 g), D-mannitol (5,000 g), low-substituted hydroxypropyl cellulose (1,200 g), and titanium oxide (Titanium Oxide NA-65: product of Toho Titanium) (240 g) were mixed, to thereby prepare a uniform powder mixture. The mixture was granulated through fluidized bed granulation in which hydroxypropylmethyl cellulose 2910 (200 g) which had been dissolved in advance in purified water (4,000 mL), and Sunset Yellow FCF (Food Yellow No. 5: product of San-Ei Gen F.F.I.) (12 g) were sprayed onto the mixture. The thus-produced granules were mixed with magnesium stearate (120 g), and the mixture was pelletized, to thereby produce scored tablets (120 mg/tablet, diameter: 7.0 mm) containing pitavastatin calcium. The scored tablets were film-coated through a conventional method employing a solution which had been prepared by dissolving hydroxypropylmethyl cellulose 2910 (480 g) and triethyl citrate (100 g) in purified water (6,000 mL) and dispersing titanium oxide (60 g) and hydrous silicon dioxide (60 g), whereby film-coated tablets (127 mg/tablet, diameter: 7.1 mm) were produced.

### Example 2

The procedure of Example 1 was repeated, except that Sunset Yellow FCF (1.2 g) and lactose (4,788.8 g) were used, to thereby prepare film-coated tablets.

### Example 3

The procedure of Example 1 was repeated, except that Sunset Yellow FCF (120 g) and lactose (4,670 g) were used, to thereby prepare film-coated tablets.

### Example 4

The procedure of Example 1 was repeated, except that titanium oxide (60 g) and lactose (4,958 g) were used, to thereby prepare film-coated tablets.

### Example 5

The procedure of Example 1 was repeated, except that titanium oxide (120 g) and lactose (4,898 g) were used, to thereby prepare film-coated tablets.

### Example 6

The procedure of Example 1 was repeated, except that titanium oxide (600 g) and lactose (4,418 g) were used, to thereby prepare film-coated tablets.

### Example 7

The procedure of Example 1 was repeated, except that titanium oxide (1,200 g) and lactose (3,818 g) were used, to thereby prepare film-coated tablets.

### Example 8

The procedure of Example 1 was repeated, except that titanium oxide was changed to titanium oxide A-HR (anatase form, product of TIOXIDE), to thereby prepare film-coated tablets.

### Example 9

Pitavastatin calcium (200 g), magnesium metasilicate aluminate (250 g), lactose (4,778 g), D-mannitol (5,000 g), and low-substituted hydroxypropyl cellulose (1,200 g) were mixed, to thereby prepare a uniform powder mixture. The mixture was granulated through fluidized bed granulation in which hydroxypropylmethyl cellulose 2910 (100 g) which had been dissolved in advance in purified water (2,000 mL), and Sunset Yellow FCF (12 g) were sprayed onto the mixture. Onto the thus-formed granules, a liquid was sprayed in a fluidized bed granulator, to thereby prepare granules to be pelletized. The liquid was prepared by dissolving hydroxypropylmethyl cellulose 2910 (100 g) in purified water (2,000 mL), and dispersing titanium oxide (240 g) in the solution. The coated granules were mixed with magnesium stearate (120 g), and the mixture was pelletized, to thereby produce scored tablets (120 mg/tablet, diameter: 7.0 mm) containing pitavastatin calcium. The scored tablets were film-coated through a conventional method employing a solution which had been prepared by dissolving hydroxypropylmethyl cellulose 2910 (480 g) and triethyl citrate (100 g) in purified water (6,000 mL) and dispersing titanium oxide (60 g) and hydrous silicon dioxide (60 g), whereby film-coated tablets (127 mg/tablet, diameter: 7.1 mm) were produced.

### Comparative Example 1

Commercial pitavastatin compound-containing pharmaceutical preparation (Livalo Tablet 2 mg (Lot: DC5A, produced and distributed by Kowa Co., Ltd.) was used.

### Comparative Example 2

The procedure of Example 1 was repeated, except that no Sunset Yellow FCF was used, to thereby prepare film-coated tablets.

### Comparative Example 3

The procedure of Example 1 was repeated, except that Sunset Yellow FCF was changed to erythrosine (maximum absorption wavelength: 526±2 nm, Food Red No. 3, product of Kishi kasei), to thereby prepare film-coated tablets.

### Comparative Example 4

The procedure of Example 1 was repeated, except that no titanium oxide was used, to thereby prepare film-coated tablets.

### Test Example

Each of the scored tablets produced in Examples 1 to 9 and Comparative Examples 1 to 4 was cut along the score line into two halves. Immediately after cutting, the tablet was irradiated with light using a day-light fluorescent lamp at a dose of 100,000 1x·h or 200,000 1x·h. The amounts (mass%) of ring-closed form 1, ring-closed form 2, and 5-keto formed through irradiation were determined. The amounts of the decomposition products were determined through HPLC (LC2010C: product of Shimadzu Corporation). Table 1 shows the results.

**Table 1**

| | | | | | | | | | | | | | | |
|---|---|---|---|---|---|---|---|---|---|---|---|---|---|---|
| Unit (%) | | | | | | | | | | | | | | |

| Component | | Examples | | | | | | | | | Comparative Examples | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Ring-closed form 1 | initial | n.d. | n.d. | n.d. | n.d. | n.d. | n.d. | n.d. | n.d. | n.d. | n.d. | n.d. | n.d. | n.d. |
| | 100,000 lx·h | 0.06 | 0.07 | 0.05 | 0.11 | 0.09 | 0.04 | 0.02 | 0.06 | 0.07 | 0.35 | 0.11 | 0.11 | 0.14 |
| | 200,000 lx·h | 0.10 | 0.10 | 0.06 | 0.13 | 0.13 | 0.05 | 0.03 | 0.07 | 0.08 | 0.46 | 0.14 | 0.16 | 0.17 |
| Ring-closed form 2 | initial | n.d. | n.d. | n.d. | n.d. | n.d. | n.d. | n.d. | n.d. | n.d. | n.d. | n.d. | n.d. | n.d. |
| | 100,000 lx·h | 0.06 | 0.07 | 0.05 | 0.10 | 0.09 | 0.04 | 0.02 | 0.06 | 0.07 | 0.34 | 0.10 | 0.11 | 0.13 |
| | 200,000 lx·h | 0.09 | 0.10 | 0.05 | 0.13 | 0.13 | 0.05 | 0.03 | 0.07 | 0.08 | 0.45 | 0.14 | 0.16 | 0.17 |
| 5-Keto form | initial | 0.04 | 0.04 | 0.04 | 0.03 | 0.03 | 0.05 | 0.05 | 0.03 | 0.02 | 0.03 | 0.04 | 0.06 | 0.03 |
| | 100,000 lx·h | 0.09 | 0.10 | 0.06 | 0.06 | 0.06 | 0.10 | 0.12 | 0.06 | 0.07 | 0.09 | 0.15 | 0.20 | 0.05 |
| | 200,000 lx·h | 0.10 | 0.12 | 0.07 | 0.07 | 0.07 | 0.12 | 0.14 | 0.07 | 0.09 | 0.11 | 0.18 | 0.24 | 0.05 |

The case in which decomposition products were detected in amounts of 0.15% or more after irradiation with light using a day-light fluorescent lamp at a dose of 200,000 lx·h was rated as instable to light.
A current pharmaceutical preparation (Comparative Example 1), a pharmaceutical preparation free of Sunset Yellow FCF (Comparative Example 2), a pharmaceutical preparation containing erythrosine (Food Red No. 3, product of Kishi kasei) instead of Sunset Yellow FCF (Comparative Example 3), and a pharmaceutical preparation containing no titanium oxide (Comparative Example 4) all exhibited, after irradiation with the light at a dose of 200,000 lx·h, at least one of the ring-closed form 1, ring-closed form 2, and 5-keto form of the corresponding pharmaceutical preparation in an amount higher than 0.15%. In other words, use of titanium oxide alone (Comparative Example 2) prevented formation of ring-closed forms, but did not prevent formation of 5-keto form. Use of Food Yellow No. 5 alone (Comparative Example 4) prevented formation of 5-keto form, but did not prevent formation of ring-closed forms. Use of titanium oxide and Food Red No. 3 in combination (Comparative Example 3) did not prevent formation of 5-keto form.
In contrast, the pharmaceutical preparations of Examples 1 to 8 all exhibited, after irradiation with the light at a dose of 200,000 lx·h, a ring-closed form 1, ring-closed form 2, and 5-keto form of the corresponding pharmaceutical preparation in an amount of 0.15% or lower, indicating excellent photostability. Among the pharmaceutical preparations of the Examples, the preparation of Example 9 exhibited the highest photostability.

### Production Example 1

Pitavastatin calcium (200 g), magnesium metasilicate aluminate (250 g), lactose (4,778 g), D-mannitol (5,000 g), and low-substituted hydroxypropyl cellulose (1,200 g) were mixed, to thereby prepare a uniform powder mixture, and the mixture was granulated through fluidized bed granulation. Onto the thus-produced granules, a liquid containing Sunset Yellow FCF (Food Yellow No. 5: product of San-Ei Gen F.F.I.) (12 g) dispersed in hydroxypropylmethyl cellulose 2910 (100 g) which had been dissolved in advance in purified water (2,000 mL) was sprayed in a fluidized bed granulator, to thereby prepare coated granules. Onto the thus-formed coated granules, a liquid was sprayed in a fluidized bed granulator, to thereby prepare granules to be pelletized, where the liquid contained hydroxypropylmethyl cellulose 2910 (100 g) which had been dissolved in advance in purified water (2,000 mL), and titanium oxide (Titanium Oxide NA-65: product of Toho Titanium) (240 g). The granules were mixed with magnesium stearate (120 g), and the mixture was pelletized, to thereby produce tablets (120 mg/tablet, diameter: 7.0 mm) containing pitavastatin calcium. The tablets were film-coated through a conventional method employing a solution which had been prepared by dissolving hydroxypropylmethyl cellulose 2910 (480 g) and triethyl citrate (100 g) in purified water (6,000 mL) and dispersing titanium oxide (60 g) and hydrous silicon dioxide (60 g) in the cellulose solution, whereby film-coated tablets (127 mg/tablet, diameter: 7.1 mm) were produced.

### Production Example 2

Pitavastatin calcium (200 g), magnesium metasilicate aluminate (250 g), lactose (4,778 g), D-mannitol (5,000 g), low-substituted hydroxypropyl cellulose (1,200 g), titanium oxide (Titanium Oxide NA-65: product of Toho Titanium) (240 g) were mixed, to thereby prepare a uniform powder mixture, and the mixture was granulated through fluidized bed granulation. Onto the thus-formed granules, hydroxypropylmethyl cellulose 2910 (200 g) which had been dissolved in advance in purified water (2,000 mL) and Sunset Yellow FCF (Food Yellow No. 5: product of San-Ei Gen F.F.I.) (12 g) were sprayed through fluidized bed granulation, to thereby prepare granules to be pelletized. The granules were mixed with magnesium stearate (120 g), and the mixture was pelletized, to thereby produce tablets (120 mg/tablet, diameter: 7.0 mm) containing pitavastatin calcium. The tablets were film-coated through a conventional method employing a solution which had been prepared by dissolving hydroxypropylmethyl cellulose 2910 (480 g) and triethyl citrate (100 g) in purified water (6,000 mL) and dispersing titanium oxide (60 g) and hydrous silicon dioxide (60 g) in the cellulose solution, whereby film-coated tablets (127 mg/tablet, diameter: 7.1 mm) were produced.

### Production Example 3

Pitavastatin calcium (200 g), magnesium metasilicate aluminate (250 g), lactose (4,778 g), D-mannitol (5,000 g), and low-substituted hydroxypropyl cellulose (1,200 g) were mixed, to thereby prepare a uniform powder mixture, and the mixture was granulated through fluidized bed granulation. Onto the thus-formed coated granules, a liquid was sprayed in a fluidized bed granulator, to thereby prepare coated granules. The liquid was prepared by dissolving hydroxypropylmethyl cellulose 2910 (100 g) in advance in purified water (2,000 mL) and dispersing titanium oxide (Titanium Oxide NA-65: product of Toho Titanium) (240 g) in the cellulose solution. Onto the thus-coated granules, a Sunset Yellow FCF (Food Yellow No. 5: product of San-Ei Gen F.F.I.) (12 g) and hydroxypropylmethyl cellulose 2910 (100 g) which had been dissolved in advance in purified water (2,000 mL) were sprayed in a fluidized bed granulator, to thereby prepare granules to be pelletized. The granules were mixed with magnesium stearate (120 g), and the mixture was pelletized to thereby produce tablets (120 mg/tablet, diameter: 7.0 mm) containing pitavastatin calcium. The tablets were film-coated through a conventional method employing a solution which had been prepared by dissolving hydroxypropylmethyl cellulose 2910 (480 g) and triethyl citrate (100 g) in purified water (6,000 mL) and dispersing titanium oxide (60 g) and hydrous silicon dioxide (60 g) in the cellulose solution, whereby film-coated tablets (127 mg/tablet, diameter: 7.1 mm were produced.

## Claims

1. A scored tablet comprising
(a) pitavastatin, a salt thereof, or an ester thereof,
(b) titanium oxide, and a
(c) colorant having a maximum absorption wavelength of 400 nm to 500 nm.

2. The scored tablet according to claim 1, which comprises granules containing pitavastatin, a salt thereof, or an ester thereof, titanium oxide, and a colorant having a maximum absorption wavelength of 400 nm to 500 nm.

3. The scored tablet according to claim 1, which comprises granules including granulated particles containing pitavastatin, a salt thereof, or an ester thereof, and a colorant having a maximum absorption wavelength of 400 nm to 500 nm, anda coating shell which contains titanium oxide and which covers each of the granulated particles.

4. The scored tablet according to claim 1, which comprises granules including granulated particles containing pitavastatin, a salt thereof, or an ester thereof, an intermediate shell which contains a colorant having a maximum absorption wavelength of 400 nm to 500 nm and which covers each of the granulated particles, anda coating shell which contains titanium oxide and which covers the intermediate shell.

5. The scored tablet according to claim 1, which comprises granules including granulated particles containing pitavastatin, a salt thereof, or an ester thereof, and titanium oxide, and a coating shell which contains a colorant having a maximum absorption wavelength of 400 nm to 500 nm and which covers each of the granulated particles.

6. The scored tablet according to claim 1, which comprises granules including granulated particles containing pitavastatin, a salt thereof, or an ester thereof, an intermediate shell which contains titanium oxide and which covers each of the granulated particles, and a coating shell which contains a colorant having a maximum absorption wavelength of 400 nm to 500 nm and which covers the intermediate shell.

7. The scored tablet according to any one of claims 1 to 6, which comprises pitavastatin, a salt thereof, or an ester thereof in an amount of 0.01 to 15 mass%, titanium oxide in an amount of 0.01 to 25 mass%, and a colorant having a maximum absorption wavelength of 400 nm to 500 nm in an amount of 0.001 to 4 mass%.

8. The scored tablet according to any one of claims 1 to 7, which comprises pitavastatin calcium.

9. The scored tablet according to any one of claims 1 to 8, wherein the colorant having a maximum absorption wavelength of 400 nm to 500 nm is Food Yellow No. 5.

10. The scored tablet according to any one of claims 1 to 10, which is a rapid-release tablet.

11. A film-coated tablet with a score line, which is obtainable by film coating the scored tablet according to any one of claims 1 to 10 with a coating liquid containing titanium oxide.

## Patentansprüche

1. Tablette mit Bruchkerbe, die
(a) Pitavastatin, ein Salz davon oder einen Ester davon,
(b) Titanoxid und
(c) einen Farbstoff mit einer maximalen Absorptionswellenlänge von 400 nm bis 500 nm,
umfasst.

2. Die Tablette mit Bruchkerbe nach Anspruch 1, die Körnchen umfasst, enthaltend Pitavastatin, ein Salz davon oder einen Ester davon, Titanoxid und einen Farbstoff mit einer maximalen Absorptionswellenlänge von 400 nm bis 500 nm.

3. Die Tablette mit Bruchkerbe nach Anspruch 1, die Körnchen umfasst, beinhaltend granulierte Teilchen, enthaltend Pitavastatin, ein Salz davon oder einen Ester davon sowie einen Farbstoff mit einer maximalen Absorptionswellenlänge von 400 nm bis 500 nm, sowie eine Beschichtungsschicht, enthaltend Titanoxid, die jedes der granulierten Teilchen bedeckt.

4. Die Tablette mit Bruchkerbe nach Anspruch 1, die Körnchen umfasst. beinhaltend granulierte Teilchen, enthaltend Pitavastatin, ein Salz davon oder einen Ester davon, eine Zwischenschicht, die einen Farbstoff mit einer maximalen Absorptionswellenlänge von 400 nm bis 500 nm enthält und die jedes der granulierten Teilchen bedeckt sowie eine Beschichtungsschicht, die Titanoxid enthält und die Zwischenschicht bedeckt.

5. Die Tablette mit Bruchkerbe nach Anspruch 1, die Körnchen umfasst, beinhaltend granulierte Teilchen, enthaltend Pitavastatin ein Salz davon oder einen Ester davon sowie Titanoxid, und eine Beschichtungsschicht, die einen Farbstoff mit einer maximalen Absorptionswellenlänge von 400 nm bis 500 nm enthält und die jedes der granulierten Teilchen bedeckt.

6. Die Tablette mit Bruchkerbe nach Anspruch 1, umfassend Körnchen, beinhaltend granulierte Teilchen, enthaltend Pitavastatin, ein Salz davon oder einen Ester davon, eine Zwischenschicht, die Titanoxid enthält und die jedes der granulierten Teilchen bedeckt sowie eine Beschichtungsschicht, die einen Farbstoff mit einer maximalen Absorptionswellenlänge von 400 nm bis 500 nm enthält, und die die Zwischenschicht bedeckt.

7. Die Tablette mit Bruchkerbe nach einem der Ansprüche 1 bis 6, die Pitavastatin, ein Salz davon oder einen Ester davon in einer Menge von 0,01 bis 15 Gewichts-%, Titanoxid in einer Menge von 0,01 bis 25 Gewichts-% sowie einen Farbstoff mit einer maximalen Absorptionswellenlänge von 400 nm bis 500 nm in einer Menge von 0,001 bis 4 Gewichts-% enthält.

8. Die Tablette mit Bruchkerbe nach einem der Ansprüche 1 bis 7, die Pitavastatinkalzium enthält.

9. Die Tablette mit Bruchkerbe nach einem der Ansprüche 1 bis 8, wobei der Farbstoff mit einer maximalen Absorptionswellenlänge 400 nm bis 500 nm Food Yellow No. 5 ist.

10. Die Tablette mit Bruchkerbe nach einem der Ansprüche 1 bis 9, die eine schnell auflösende Tablette ist.

11. Filmbeschichtete Tablette mit einer Bruchkerbe, die erhältlich ist durch Beschichten der Tablette mit Bruchkerbe nach einem der Ansprüche 1 bis 10 mit einer Beschichtungsflüssigkeit, die Titanoxid enthält.

## Revendications

1. Comprimé sécable comprenant :
(a) de la pitavastatine, un sel de celle-ci, ou un ester de celle-ci,
(b) de l'oxyde de titane, et
(c) un colorant ayant une longueur d'onde d'absorption maximale de 400 nm à 500 nm.

2. Comprimé sécable selon la revendication 1, qui comprend des granules contenant de la pitavastatine, un sel de celle-ci, ou un ester de celle-ci, de l'oxyde de titane, et un colorant ayant une longueur d'onde d'absorption maximale de 400 nm à 500 nm.

3. Comprimé sécable selon la revendication 1, qui comprend des granules contenant des particules granulées contenant de la pitavastatine, un sel de celle-ci, ou un ester de celle-ci, et un colorant ayant une longueur d'onde d'absorption maximale de 400 nm à 500 nm, et une coquille d'enrobage qui contient de l'oxyde de titane et qui recouvre chacune des particules granulées.

4. Comprimé sécable selon la revendication 1, qui comprend des granules contenant des particules granulées contenant de la pitavastatine, un sel de celle-ci, ou un ester de celle-ci, une coquille intermédiaire qui contient un colorant ayant une longueur d'onde d'absorption maximale de 400 nm à 500 nm et qui recouvre chacune des particules granulées, et une coquille d'enrobage qui contient de l'oxyde de titane et qui recouvre la coquille intermédiaire.

5. Comprimé sécable selon la revendication 1, qui comprend des granules contenant des particules granulées contenant de la pitavastatine, un sel de celle-ci, ou un ester de celle-ci, et de l'oxyde de titane, et une coquille d'enrobage qui contient un colorant ayant une longueur d'onde d'absorption maximale de 400 nm à 500 nm et qui recouvre chacune des particules granulées.

6. Comprimé sécable selon la revendication 1, qui comprend des granules contenant des particules granulées contenant de la pitavastatine, un sel de celle-ci, ou un ester de celle-ci, une coquille intermédiaire qui contient de l'oxyde de titane et qui recouvre chacune des particules granulées, et une coquille d'enrobage qui contient un colorant ayant une longueur d'onde d'absorption maximale de 400 nm à 500 nm et qui recouvre la coquille intermédiaire.

7. Comprimé sécable selon l'une quelconque des revendications 1 à 6, qui comprend de la pitavastatine, un sel de celle-ci, ou un ester de celle-ci, en une quantité de 0,01 à 15 % en masse, de l'oxyde de titane en une quantité de 0,01 à 25 % en masse, et un colorant ayant une longueur d'onde d'absorption maximale de 400 nm à 500 nm en une quantité de 0,001 à 4 % en masse.

8. Comprimé sécable selon l'une quelconque des revendications 1 à 7, qui comprend de la pitavastatine calcique.

9. Comprimé sécable selon l'une quelconque des revendications 1 à 8, dans lequel le colorant ayant une longueur d'onde d'absorption maximale de 400 nm à 500 nm est le Food Yellow N° 5.

10. Comprimé sécable selon l'une quelconque des revendications 1 à 9, qui est un comprimé à libération rapide.

11. Comprimé pelliculé avec un trait de coupe, qui peut être obtenu par pelliculage du comprimé sécable selon l'une quelconque des revendications 1 à 10 avec un liquide d'enrobage contenant de l'oxyde de titane.
